# EUROPEAN PATENT SPECIFICATION

(11) **EP 3 808 729 B1**
(45) Date of publication and mention of the grant of the patent: **10.12.2025**
(21) Application number: 20200615.1
(22) Date of filing: 07.10.2020
(51) Int. Cl.: C07C 67/08, C07C 67/14, C07C 69/30, C07C 69/58

(54) **METHOD FOR PRODUCING 1-PALMITOYL-2-LINOLEOYL-3-ACETYL GLYCEROL**
VERFAHREN ZUR HERSTELLUNG VON 1-PALMITOYL-2-LINOLEOYL-3-ACETYLGLYCEROL
PROCÉDÉ DE PRODUCTION DE 1-PALMITOYL-2-LINOLEOYL-3-ACÉTYL GLYCÉROL

(30) Priority: 15.10.2019 KR 20190128021
(43) Date of publication of application: 21.04.2021
(73) Proprietor: Enzychem Lifesciences Corporation, Chungcheongbuk-do 27159 (KR)
(72) Inventor: SOHN, Ki Young, Seoul 07264 (KR); KANG, Byung Kyu, Chungcheongbuk-do 27109 (KR)
(74) Representative: IPAZ

(56) References cited:
- EP-A2- 2 759 529
- EP-B1- 2 759 529
- US-A1- 2015 266 803

## Description

### Cross-reference to Related Application

This application claims benefit of priority to Korean Patent Application No. 10-2019-0128021 filed on October 15, 2019.

### Field of Invention

This invention relates to a method for producing 1-palmitoyl-2-linoleoyl-3-acetyl glycerol, and more particularly, to a method for producing 1-palmitoyl-2-linoleoyl-3-acetyl glycerol of high purity with high yield by carrying out acetylation of 1-palmitoyl glycerol with acetic anhydride or a mixture of acetic anhydride and acetyl chloride.

### Background of Invention

Rac-1-palmitoyl-2-linoleoyl-3-acetyl glycerol is one of components of deer antler, is separated from chloroform extracts of deer antler, and shows the growth-stimulating effects of hematopoietic stem cells and megakaryocytes (Korean Patent No. 10-0283010). Chemical synthesis methods of rac-1-palmitoyl-2-linoleoyl-3-acetyl glycerol are disclosed in Korean Patent Nos. 10-0291743 and 10-1278874.

In Korean Patent No. 10-0291743, two methods for preparing the rac-1-palmitoyl-2-linoleoyl-3-acetyl glycerol, (a) a method of synthesizing the compound from glycerol and (b) a method of synthesizing the compound from acetolysis of phosphatidylcholine are disclosed. According to (a) the method of synthesizing the compound from glycerol, 1-palmitoyl glycerol is separated with a column chromatography from reaction product of glycerol and palmitic acid, the separated 1-palmitoylglycerol is acetylated and then the acetylated product is separated again with a column chromatography. Then, a linoleoylation reaction is carried out for the above reaction product and a further separation step with a column chromatography is carried out to produce the target compound of rac-1-palmitoyl-2-linoleoyl-3-acetyl glycerol. In said method, there is no regioselectivity for starting materials and intermediates, thus the reaction product of each reaction step should be separated and purified with a column chromatography, and the overall yield of the target compound is very low (about 3.21% from glycerol). Furthermore, in said method, since an esterification reaction (Steglich esterification) using N,N- Cyclohexylcarboimide (DCC) is carried out, a side reaction, in which an acyl group is migrated in an adduct of DCC and carboxylic acid, may occur. To reduce the side reaction, an expensive catalyst, 4-dimethylaminopyridine(DMAP) should be used in the amount of more than one equivalent. However, the side reaction cannot be completely suppressed, dicyclohexylurea is formed as a by-product, and it is difficult to completely remove the by-product by a filtration and an extraction.

In Korean Patent No. 10-1278874 and in its US counterpart patent application No. US2015/266803, 1-palmitoylglycerol and an acetylating agent are reacted to produce an acetylation product, and the obtained acetylation product is crystallized to separate 1-palmitoyl-3-acetyl glycerol. Subsequently, a linoleoyl group is introduced into the separated 1-palmitoyl-3-acetyl glycerol to produce rac-1-palmitoyl-2-linoleoyl-3-acetyl glycerol. In the above document, acetyl chloride or acetyl bromide is used as the acetylating agent. However, when acetyl chloride or acetyl bromide is used as the acetylating agent, strong acids such as hydrochloric acid or bromic acid are produced as reaction by-products, the stability decreases as the reaction time increases, and the reaction product may be decomposed. In this case, the reaction yield can be lowered, and an additional purification step can be required to improve the purity of the product.

### Summary of invention

It is an object of the present invention to provide a method for producing 1-palmitoyl-2-linoleoyl-3-acetyl glycerol which suppresses the formation of impurities or degradation products and does not need an additional purification step.

It is another object of the present invention to provide a method for producing 1-palmitoyl-2-linoleoyl-3-acetyl glycerol of high purity with high yield.

In order to achieve these and other objects, the present invention provides a method for producing 1-palmitoyl-2-linoleoyl-3-acetyl glycerol of the following Chemical formula 3, comprising the steps of: preparing 1-palmitoyl-3-acetyl glycerol intermediate of the following Chemical formula 2 by reacting 1-palmitoyl glycerol of the following Chemical formula 1 and an acetylating agent of a mixture of acetic anhydride and acetyl chloride; and reacting the 1-palmitoyl-3-acetyl glycerol intermediate and linoleic acid, 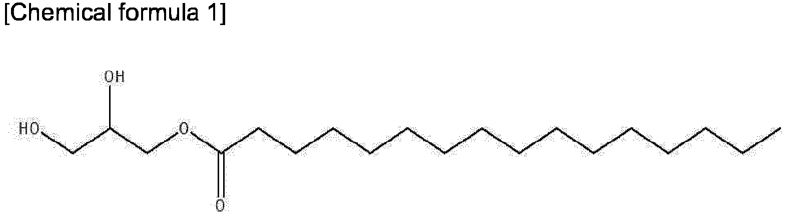 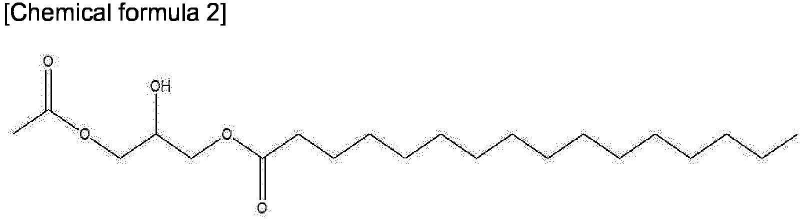 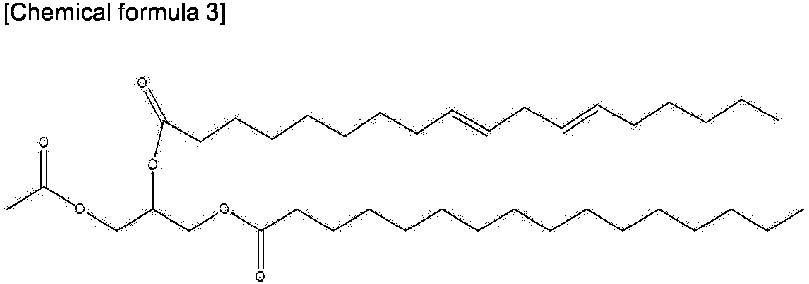 wherein the amount of the mixture of acetic anhydride and acetyl chloride is 1.1 to 1.3 equivalent with respect to 1-palmitoyl glycerol, and a ratio of acetic anhydride and the acetyl chloride is 1:2 to 1:2.5 in equivalent ratio, and a reaction temperature of 1-palmitoyl glycerol and the acetylating agent is 5 to 15°C.

The method for producing 1-palmitoyl-2-linoleoyl-3-acetyl glycerol according to the present invention suppresses the formation of impurities or degradation products and does not need additional purification step, and produces 1-palmitoyl-2-linoleoyl-3-acetyl glycerol of high purity with high yield.

### Detailed Description of Invention

Hereinafter, the present invention will be explained in more detail.

For preparing 1-palmitoyl-2-linoleoyl-3-acetyl glycerol according to the present invention, firstly, 1-palmitoyl glycerol of the following Chemical formula 1 is reacted with an acetylating agent of a mixture of acetic anhydride and acetyl chloride to prepare 1-palmitoyl-3-acetyl glycerol intermediate of the following Chemical formula 2, wherein the amount of the mixture of acetic anhydride and acetyl chloride is 1.1 to 1.3 equivalent with respect to 1-palmitoyl glycerol, and a ratio of acetic anhydride and the acetyl chloride is 1:2 to 1:2.5 in equivalent ratio, and a reaction temperature of 1-palmitoyl glycerol and the acetylating agent is 5 to 15°C.

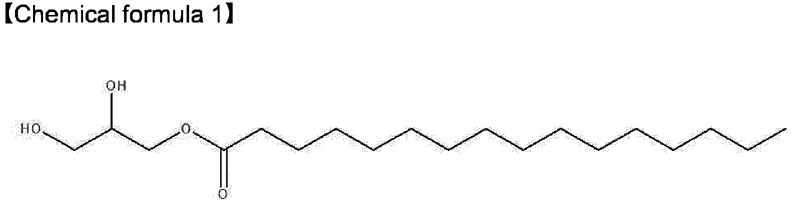

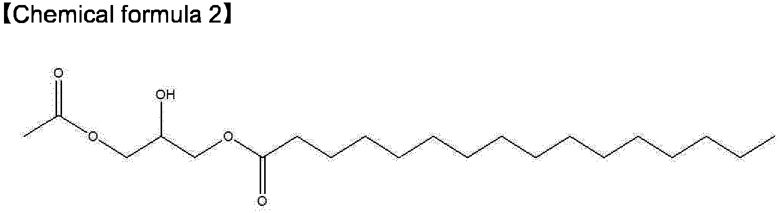

In the present invention, a mixture of acetic anhydride and acetyl chloride is used as the acetylating agent, and thereby decomposition products are not produced or the amount of decomposition products can be decreased, and an additional purification process is not necessary. In detail, when a conventional acetylating agent such as acetyl chloride alone is used, the palmitoyl group of 1-palmitoyl glycerol or acetyl group is decomposed by hydrochloric acid generated from acetyl chloride. Thus, an additional purification process to remove the decomposed palmitoyl group, acetyl group, etc. should be carried out. However, according to the present invention, 1-palmitoyl glycerol is acetylated with a mixture of acetic anhydride and acetyl chloride, so that decomposition of the palmitoyl group and acetyl group of 1-palmitoyl glycerol can be suppressed. The process of purifying the decomposed palmitoyl group, acetyl group, etc. is not necessary, and 1-palmitoyl-3-acetyl glycerol intermediate of high purity can be obtained. Therefore, according to the present invention, since an additional purification process for purifying the intermediate is not necessary, the process time and manufacturing cost can be reduced.

The amount of the mixture of acetic anhydride and acetyl chloride is 1.1 to 1.3 equivalent with respect to 1-palmitoyl glycerol. If the amount of the mixture of acetic anhydride and acetyl chloride is less than 0.7 equivalent, unreacted material may remain, and if it is more than 2.0 equivalent, 1-palmitoyl-2,3-acetyl glycerol may be generated as a by-product. In the mixture of acetic anhydride and acetyl chloride, ratio of the amount of acetic anhydride and the amount of acetyl chloride is in an equivalent ratio of 1:2 to 1:2.5. When the amount of acetyl chloride to acetic anhydride is less than 1.5 equivalent ratio, unreacted products may remain, and when the amount ratio of acetyl chloride to acetic anhydride exceeds 3.5 equivalent ratio, by-products may increase.

The acetylation reaction can be carried out in a solvent and in the presence of an organic base and a catalyst. Examples of the organic base include pyridine and so on. The amount of the organic base is 1.5 to 7.0 equivalents, preferably 3 to 5 equivalents with respect to 1-palmitoyl glycerol. When the amount of the organic base is less than 1.5 equivalents with respect to 1-palmitoyl glycerol, acids produced during the acetylation reaction may not be fully neutralized. When the amount of the organic base is more than 7 equivalents with respect to 1-palmitoyl glycerol, there is no additional advantage. As the catalyst, Dimethylaminopyridine (DMAP) and so on may be used, and the amount of the catalyst is 0.005 to 0.05 equivalent, preferably 0.01 to 0.03 equivalent with respect to 1-palmitoyl glycerol. If the amount of the catalyst is less than 0.005 equivalent, the reaction rate may be slow, and if it exceeds 0.05 equivalent, there is no special advantage. Examples of the solvent include a non-polar and aprotic solvent such as dichloromethane(methylene chloride), acetone, ethyl acetate, the mixtures thereof, and so on, and preferably include dichloromethane. The amount of the solvent is 5 to 10 times, preferably 6 to 8 times in volume ratio with respect to the weight of 1-palmitoylglycerol (volume/weight). When the amount of the solvent is too small, the reaction mixture may not be smoothly stirred due to the salt precipitated during the reaction. When the amount of the solvent is too much, there is no additional advantage.

The reaction temperature of the acetylation is 5 to 15 °C, the reaction time is 5 to 10 hours, preferably 6 to 8 hours. Here, if the reaction temperature is too low or the reaction time is too short, there is a risk that acetylation may not proceed sufficiently, and if the reaction temperature is too high or the reaction time is too long, by-products may be generated or the product may be decomposed. When the acetylation is completed, if necessary, 1-palmitoyl-2,3-diacetyl glycerol (1-PAA), a by-product which might be produced by reaction of 1-palmitoyl glycerol (1-PG) and two acetyl groups, can be purified and removed.

Next, the 1-palmitoyl-3-acetyl glycerol intermediate and linoleic acid are reacted to obtain 1-palmitoyl-2-linoleoyl-3-acetyl glycerol of the following Chemical Formula 3.

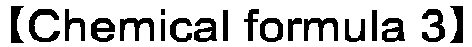

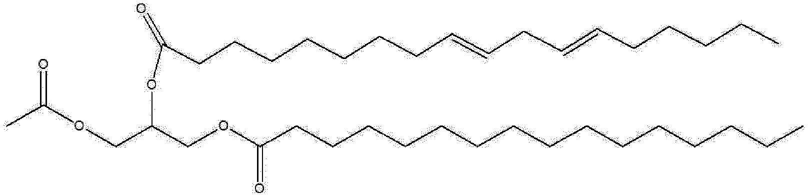

The linoleic acid can be preferably used in the form of a mixed anhydride 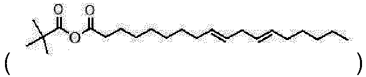 which is an activated form of linoleic acid and produced by reacting linoleic acid and pivaloyl chloride. The reaction of linoleic acid and pivaloyl chloride may be performed in an organic solvent in the presence of an organic base. Examples of the organic solvent may include saturated hydrocarbon solvents having 5 to 7 carbon atoms such as pentane, hexane, heptane, and mixtures thereof. The organic base is for neutralizing hydrochloric acid generated in the reaction of linoleic acid and pivaloyl chloride, and for neutralizing pivalic acid generated in the reaction of the prepared mixed anhydride and 1-palmitoyl-3-acetyl glycerol. Examples of the organic base may include triethylamine (TEA) and so on.

The reaction of 1-palmitoyl-3-acetyl glycerol intermediate and linoleic acid may be carried out in an organic solvent in the presence of a catalyst. As the catalyst, Dimethylaminopyridine (DMAP), etc. can be used. The amount of the catalyst used is 0.01 to 0.2 equivalent, preferably 0.05 to 0.15 equivalent with respect to 1-palmitoyl-3-acetyl glycerol. If the amount of the catalyst is less than 0.01 equivalent, the reaction rate may decrease, and even if it exceeds 0.2 equivalent, there is no special advantage. As the organic solvent, a saturated hydrocarbon solvent having 5 to 7 carbon atoms, such as pentane, hexane, heptane, or a mixture thereof can be used.

The reaction temperature of 1-palmitoyl-3-acetyl glycerol intermediate and linoleic acid is 20 to 40 °C, preferably 25 to 35 °C, the reaction time is 4 to 10 hours, preferably 5 to 6 hours. If the reaction temperature is less than 20 °C, the reaction rate may be slowed, and if the reaction temperature exceeds 40 °C, by-products may be generated. In addition, if the reaction time is less than 4 hours, the reaction may not be completed, and even if it exceeds 10 hours, there is no special advantage.

Hereinafter, examples of the present invention are provided for better understanding of the present invention. The following examples are exemplary, and the present invention is not limited by the examples.

### [Example 1] Preparation of 1-palmitoyl-3-acetyl glycerol intermediate

To a 250 mL 3-neck round flask, 20 g of 1-palmitoyl glycerol, 140 mL of dichloromethane, 33.5 g of pyridine and 0.15 g of dimethylaminopyridine were added, and the mixture was heated to 30 °C to dissolve 1-palmitoyl glycerol. 3.1 g of acetic anhydride was added dropwise while maintaining the temperature of the reactant at 18 °C, and 5.7 g of acetyl chloride and 11 mL of dichloromethane were added dropwise while maintaining the temperature at 5 °C, and the reaction was carried out for 18 hours. After the reaction was completed, 90 mL of purified water and 40 g of concentrated hydrochloric acid were added, the temperature was raised to 25 °C, and the aqueous layer was separated to obtain an organic layer. The obtained organic layer was further subjected to a layer separation process using purified water once more. Magnesium sulfate and potassium carbonate were added to the separated organic layer, stirred for 1 hour, and then magnesium sulfate and potassium carbonate were filtered off and the filtrate was concentrated. 40 mL of normal hexane was added to the filtrate and concentrated again, and 60 mL of normal hexane was additionally added and cooled to 10 °C. When a solid was formed, the reactant was stirred for 2 hours while maintaining the temperature, and the resulting solid was filtered and dried to obtain 20 g of 1-palmitoyl-3-acetyl glycerol in the form of a white solid (yield: 88.7 %).

### [Example 2] Preparation of 1-palmitoyl-3-acetyl glycerol intermediate (Example not forming part of the present invention)

To a 250 mL 3-neck round flask, 30 g of 1-palmitoyl glycerol, 180 mL of dichloromethane, 50.3 g of pyridine and 0.22 g of dimethylaminopyridine were added, acetic anhydride was added dropwise while maintaining the temperature of the reactant at 18 °C. After the dropwise addition, the temperature was maintained at 5 °C, and the reaction was carried out for 22 hours.

After the reaction was completed, 90 mL of purified water and 40 g of concentrated hydrochloric acid were added, the temperature was raised to 25 °C, and the aqueous layer was separated to obtain an organic layer. The obtained organic layer was further subjected to a layer separation process using purified water once more. Magnesium sulfate and potassium carbonate were added to the separated organic layer, stirred for 1 hour, and then magnesium sulfate and potassium carbonate were filtered off and the filtrate was concentrated. 40 mL of normal hexane was added to the filtrate and concentrated again, and 60 mL of normal hexane was additionally added and cooled to 10 °C. When a solid was formed, the mixture was stirred for 2 hours while maintaining the temperature, and the resulting solid was filtered and dried to obtain 29 g of 1-palmitoyl-3-acetyl glycerol in the form of a white solid (yield: 85.8 %).

### [Example 3] Preparation of 1-palmitoyl-2-linoleoyl-3-acetyl glycerol

To a 250 mL 3-neck round flask, 7.67 g of linoleic acid, 80 mL of normal hexane and 3.31 g of pivaloyl chloride were added, and the reactant was cooled to -5 to 10 °C and 5.97 g of triethylamine was added dropwise. After adding 10 g of 1-palmitoyl-3-acetyl glycerol and 0.328 g of 4-dimethylaminopyridine to the reactant, the reactant was reacted for 12 hours while stirring under nitrogen gas.

After the reaction was completed, 40 mL of purified water was added to the reactant, and the aqueous layer was layer-separated to obtain a washed organic layer. To the separated organic layer, a mixed solution of 27 mL of methanol and 13.5 mL of 0.1N KOH was added, washed twice, and further washed with 40 mL of a mixed solution of 95 vol% methanol purified water, washed with 10 mL of 0.1 vol% hydrochloric acid, and then washed with 40 mL of an aqueous 0.05 weight% sodium hydrogen carbonate solution. 8 g of activated carbon, 10 g of magnesium sulfate and 10 g of clay were added to the washed organic layer and the organic layer was stirred for 2 hours while maintaining 0 to 5°C. Then the activated carbon was filtered and the organic layer was washed with normal hexane which is cooled to -10 °C to obtain 8.5 g of 1-palmitoyl-2-linoleoyl-3-acetyl glycerol in the form of a pale yellow oil (Yield: 74.8%).

## Claims

1. A method for producing 1-palmitoyl-2-linoleoyl-3-acetyl glycerol of the following Chemical formula 3, comprising the steps of:
preparing 1-palmitoyl-3-acetyl glycerol intermediate of the following Chemical formula 2 by reacting 1-palmitoyl glycerol of the following Chemical formula 1 and an acetylating agent of a mixture of acetic anhydride and acetyl chloride; and
reacting the 1-palmitoyl-3-acetyl glycerol intermediate and linoleic acid, 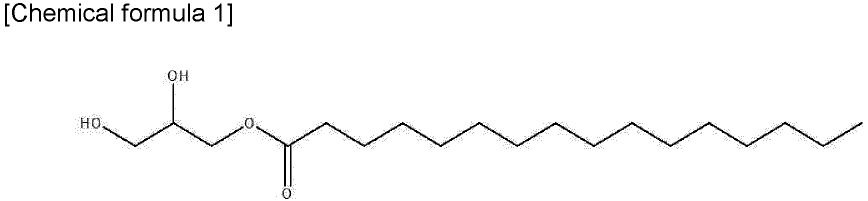 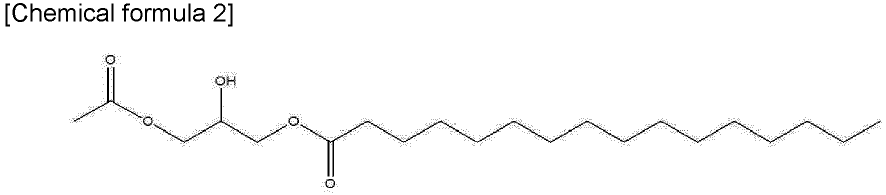 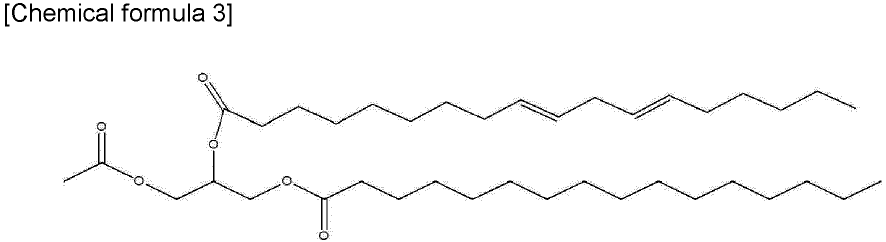
wherein the amount of the mixture of acetic anhydride and acetyl chloride is 1.1 to 1.3 equivalent with respect to 1-palmitoyl glycerol, and a ratio of acetic anhydride and the acetyl chloride is 1:2 to 1:2.5 in equivalent ratio, and
a reaction temperature of 1-palmitoyl glycerol and the acetylating agent is 5 to 15°C.

2. The method for producing 1-palmitoyl-2-linoleoyl-3-acetyl glycerol according to claim 1, wherein the 1-palmitoyl-3-acetyl glycerol intermediate is obtained without purifying decomposed palmitoyl group from reaction of 1-palmitoyl glycerol and the acetylating agent.

3. The method for producing 1-palmitoyl-2-linoleoyl-3-acetyl glycerol according to claim 1, wherein the reaction of 1-palmitoyl glycerol and the acetylating agent is carried out in the presence of an organic base and a catalyst, the organic base is pyridine, and the catalyst is dimethylaminopyridine.

## Patentansprüche

1. Verfahren zum Herstellen von 1-Palmitoyl-2-linoleoyl-3-acetylglycerin der folgenden chemischen Formel 3, umfassend die Schritte:
Vorbereiten eines 1-Palmitoyl-3-acetylglycerin-Zwischenprodukts der folgenden chemischen Formel 2 durch Reagieren von 1-Palmitoylglycerin der folgenden chemischen Formel 1 und einem Acetylierungsmittel aus einer Mischung von Essigsäureanhydrid und Acetylchlorid; und
Reagieren des 1-Palmitoyl-3-Acetylglycerin-Zwischenprodukts und Linolsäure, 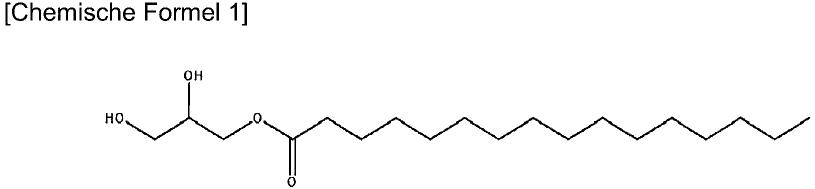 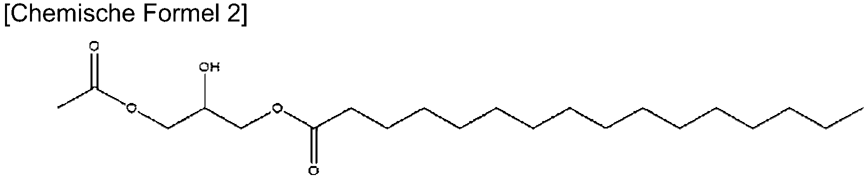 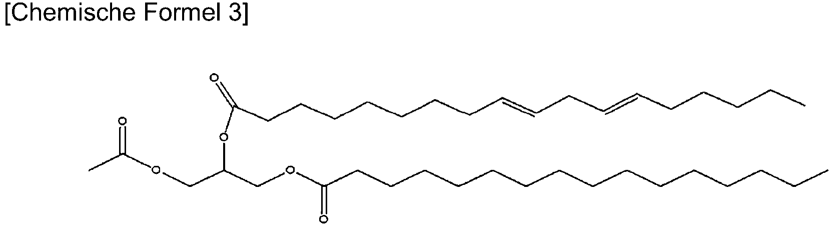 wobei die Menge der Mischung aus Essigsäureanhydrid und Acetylchlorid 1,1 bis 1,3 Äquivalente in Bezug auf 1-Palmitoylglycerin ist und ein Verhältnis von Essigsäureanhydrid und Acetylchlorid 1:2 bis 1:2,5 im Äquivalentverhältnis ist, und eine Reaktionstemperatur von 1-Palmitoylglycerol und dem Acetylierungsmittel 5 bis 15 °C beträgt.

2. Verfahren zum Herstellen von 1-Palmitoyl-2-linoleoyl-3-acetylglycerin nach Anspruch 1, wobei das 1-Palmitoyl-3-acetylglycerin-Zwischenprodukt ohne Reinigen der zersetzten Palmitoylgruppe aus der Reaktion von 1-Palmitoylglycerin und dem Acetylierungsmittel erhalten wird.

3. Verfahren zum Herstellen von 1-Palmitoyl-2-linoleoyl-3-acetylglycerin nach Anspruch 1, wobei die Reaktion von 1-Palmitoylglycerin und dem Acetylierungsmittel in Gegenwart einer organischen Base und eines Katalysators vorgenommen wird, wobei die organische Base Pyridin und der Katalysator Dimethylaminopyridin ist.

## Revendications

1. Procédé de production de 1-palmitoyl-2-linoléoyl-3-acétyl glycérol de formule chimique 3 suivante, comprenant les étapes suivantes :
préparation de l'intermédiaire 1-palmitoyl-3-acétyl glycérol de formule chimique 2 suivante par réaction du 1-palmitoyl glycérol de formule chimique 1 suivante et d'un agent d'acétylation constitué d'un mélange d'anhydride acétique et de chlorure d'acétyle ; et
réaction de l'intermédiaire 1-palmitoyl-3-acétyl glycérol et de l'acide linoléique, 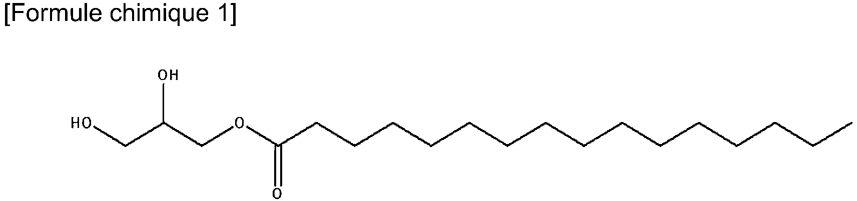 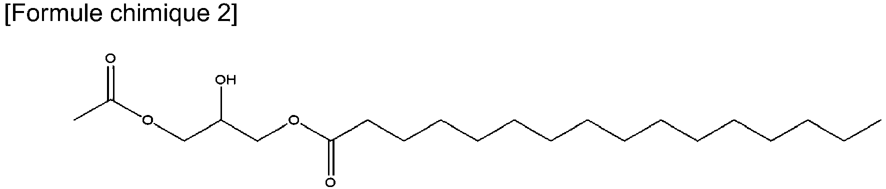 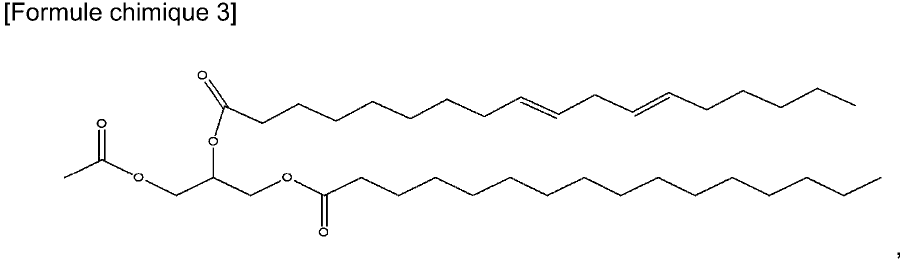
dans lequel la quantité du mélange d'anhydride acétique et de chlorure d'acétyle est comprise entre 1,1 et 1,3 équivalents par rapport au 1-palmitoylglycérol, et le rapport entre l'anhydride acétique et le chlorure d'acétyle est compris entre 1:2 et 1:2,5 en équivalents, et
la température de réaction du 1-palmitoylglycérol et de l'agent d'acétylation est comprise entre 5 et 15°C.

2. Procédé de production de 1-palmitoyl-2-linoléoyl-3-acétyl glycérol selon la revendication 1, dans lequel l'intermédiaire 1-palmitoyl-3-acétyl glycérol est obtenu sans purifier le groupe palmitoyle décomposé issu de la réaction du 1-palmitoyl glycérol et de l'agent acétylant.

3. Procédé de production de 1-palmitoyl-2-linoléoyl-3-acétyl glycérol selon la revendication 1, dans lequel la réaction du 1-palmitoyl glycérol et de l'agent acétylant est effectuée en présence d'une base organique et d'un catalyseur, la base organique est la pyridine et le catalyseur est la diméthylaminopyridine.
